# EUROPÄISCHE PATENTSCHRIFT

(11) **EP 1 769 823 B1**
(45) Veröffentlichungstag und Bekanntmachung des Hinweises auf die Patenterteilung: **22.04.2009**
(21) Anmeldenummer: 06018401.7
(22) Anmeldetag: 02.09.2006
(51) Int. Cl.: A61N 1/39, A61B 5/046, A61N 1/362, A61N 1/365

(54) **Detektor für atriates Flimmern und Flattern**
Detector for atrial fibrillation and atrial flutter
Détecteur de la fibrillation auriculaire et du flutter auriculaire

(30) Priorität: 30.09.2005 DE 102005047320
(43) Veröffentlichungstag der Anmeldung: 04.04.2007
(73) Patentinhaber: BIOTRONIK CRM Patent AG, 6341 Baar (CH); Universitätsklinikum Freiburg, 79106 Freiburg (DE)
(72) Erfinder: S. Faber, Thomas, Dr., 79104 Freiburg (DE); Lippert, Michael, Dr., 91522 Ansbach (DE); Schweika-Kresimon, Marc Oliver, Dr., 44625 Herne (DE)
(74) Vertreter: Lindner-Vogt, Karin L.

(56) Entgegenhaltungen:
- EP-A- 0 583 499
- EP-A- 1 384 433
- WO-A-20/04028629
- US-A- 5 720 295

## Beschreibung

Die Erfindung betrifft einen Detektor für atriales Flimmern, auch als atriale Fibrillation bekannt, und atriales Flattern ("AF" steht im Folgenden für atriales Flimmern oder Flattern). Der Detektor ist vorzugsweise Bestandteil eines medizinischen Implantats wie beispielsweise eines implantierbaren Herzschrittmachers oder eines implantierbaren Kardioverters/Defibrillators.

Die atriale Fibrillation ist ein Zustand ungeordneter, das atriale Flattern beispielsweise kreisender Erregung, des atrialen Myokards, bei dem das Atrium praktisch keinen Beitrag zur Pumpleistung eines Herzens leistet. In einem intraatrialen Elektrokardiogramm ist eine atriale Fibrillation an einer hohen Erregungsfrequenz und geringer Amplitude zu erkennen.

Es ist bekannt, eine atriale Defibrillation beispielsweise mit Hilfe implantierbarer atrialer Defibrillatoren zu behandeln. Beispiele für derartige atriale Defibrillatoren und Detektoren für atriale Fibrillation sind in US 5,267,559, US 5,433,729, US 5,464,431, US 5,464,432, US 5,486,199 und US 5,720,295 zu finden.

Vor dem Hintergrund des bekannten Standes der Technik ist es die Aufgabe der Erfindung, einen Detektor zur zuverlässigen Erkennung von AF zu schaffen, der sich mit vertretbarem Aufwand realisieren lässt und der eine möglichst hohe Sensitivität sowie auch eine möglichst hohe Spezifität besitzt.

Erfindungsgemäß wird diese Aufgabe durch einen Detektor für AF gelöst, der eine Impedanzmesseinheit aufweist, welche einen Messeingang besitzt, an dem eine atriale Elektrodenleitung mit einer Elektrode für eine unipolare Messung einer Impedanz im Atrium anzuschließen ist oder angeschlossen ist. Die Impedanzmesseinheit ist ausgebildet, ein unipolar gewonnenes, atriales Impedanzsignal derart zu generieren, dass das Impedanzsignal für jeden, eine atriale Kontraktion und die darauf folgende Entspannung des Atriums umfassenden atrialen Zyklus mehrere, zu verschiedenen Zeitpunkten innerhalb eines jeweiligen atrialen Zyklusses erfasste Impedanzwerte umfasst. Mit anderen Worten: im Betrieb tastet die Impedanzmesseinheit die atriale Impedanz mit einer Frequenz ab, die größer ist, als die durch die Dauer des atrialen Zyklus (AA-Intervall) bestimmte, atriale Kontraktionsrate.

Das dazu ausgewertete unipolare atriale Impedanzsignal ist eine Impedanz, die zwischen einer Neutralelektrode, beispielsweise dem Gehäuse eines implantierbaren Defibrillators oder Herzschrittmachers, und einer kleinflächigen, wandnahen Elektrode im Atrium gemessen ist. Vorzugsweise erfolgt zur Bestimmung dieses unipolaren atrialen Impedanzsignals die Einspeisung eines konstanten, gepulsten Messstroms über dieselben beiden Elektroden, beispielsweise eine atriale Tip-Elektrode und das Schrittmacher- oder Defibrillatorgehäuse, über die gleichzeitig auch ein in Folge der Stromimpulse auftretender Spannungsabfall gemessen wird.

Neben der Impedanzmesseinheit weist der Detektor außerdem einen Signaleingang auf, über den dem Detektor ein Ventrikelsignal zuzuführen ist, welches die Zeitpunkte ventrikulärer Kontraktionen in zeitlicher Zuordnung zu dem atrialen Impedanzsignal widerspiegelt. Ein solches Ventrikelsignal kann ein intraventrikuläres Elektrokardiogramm sein, es kann aber auch ein aus diesem abgeleitetes Signal wie beispielsweise das Signal eines ventrikulären Markerkanals sein. In allen Fällen ist das Ventrikelsignal ein Signal, welches die Zeitpunkte ventrikulärer Kontraktionen wiedergibt, die sich als R-Zacken im intraventrikulären Elektrokardiogramm widerspiegeln. Für die Erfindung ist es nicht entscheidend, ob das Ventrikelsignal ein Rohsignal ist, wie es unmittelbar durch Erfassen elektrischer Potentiale im Ventrikel gewonnen wird (also ein EKG) oder ein daraus abgeleitetes Signal wie das Signal eines Markerkanals ist.

Weiterhin weist der Detektor neben der Impedanzmesseinheit und dem Signaleingang für das Ventrikelsignal eine Auswerteeinheit auf, die ausgebildet,
mehrere aufeinanderfolgende, von jeweils zwei aufeinander folgenden ventrikulären Kontraktionen begrenzte Impedanzsignalabschnitte eines unipolaren atrialen Impedanzsignals miteinander zu mitteln und
die maximale Amplitude des gemittelten unipolaren atrialen Impedanzsignalabschnitts zu bestimmen,
mit einem Vergleichswert zu vergleichen, und
im Falle, dass die maximale Amplitude des gemittelten unipolaren atrialen Impedanzsignal geringer ist, als der Vergleichswert, ein AF-Verdachtsignal zu erzeugen.

Das AF-Verdachtssignal kennzeichnet einen atrialen Zustand, in dem der Verdacht auf eine atriale Fibrillation oder atriales Flattern besteht.

Die Mittelung des atrialen Impedanzverlaufes über mehrere ventrikuläre Zyklen erfolgt dabei in keiner Weise derart, dass das atriale Impedanzsignal im Falle unterschiedlich langer ventrikulärer Zyklen gestaucht oder gestreckt wird. Vielmehr werden entweder ein Anfangs- oder ein Endabschnitt eines jeweiligen atrialen Impedanzabschnittes, der länger ist, als ein kürzester der zu mittelnden atrialen Impedanzabschnitte, für die Mittelung außer Betracht gelassen.

Die Erfindung beruht auf der Erkenntnis, dass im Falle von AF atriale Kontraktionen und ventrikuläre Kontraktionen derart dissoziiert sind, dass zwischen den atrialen und den ventrikulären Kontraktionen nicht die bei gesunden Herzen vorhandene Synchronizität besteht. Dies führt dazu, dass die atrialen Kontraktionen bezogen auf aufeinanderfolgende, ventrikuläre Zyklen zu unterschiedlichen Zeitpunkten innerhalb eines jeweiligen ventrikulären Zyklus auftreten. Wenn somit das atriale Impedanzsignal über mehrere ventrikuläre Zyklen zyklusweise (bezogen auf die ventrikulären Kontraktionen) gemittelt wird, mitteln sich noch vorhandene Spitzenamplituden des atrialen Impedanzsignals so weitgehend aus, dass das gemittelte unipolare Impedanzsignal keine Spitzenamplituden nennenswerter Größe mehr aufweist. Dieser Zustand wird erfindungsgemäß dadurch erfasst, dass das gemittelte unipolare Impedanzsignal mit einem vorgegebenen, gegebenenfalls automatisch anpassbaren Vergleichswert verglichen wird. Wenn die Spitzenamplitude des gemittelten unipolaren atrialen Impedanzsignals kleiner ist, als der Vergleichswert, liegt mit großer Wahrscheinlichkeit AF vor. In diesem Fall wird ein AF-Verdachtssignal erzeugt.

In einer bevorzugten Ausführungsvariante ist die Auswerteeinheit ausgebildet, immer die jeweils acht aktuellsten, zwischen zwei aufeinander folgenden ventrikulären Kontraktionen liegenden Abschnitte des atrialen Impedanzsignals miteinander zu mitteln. Es hat sich herausgestellt, dass bereits eine Mittlung über acht ventrikuläre Zyklen zu einer ausreichend signifikanten Reduktion der Spitzenamplitude des atrialen Impedanzsignals führt. Außerdem lässt sich auf diese Weise bereits nach acht ventrikulären Zyklen AF feststellen. Die Mittelung kann auch über mehr als acht ventrikuläre Zyklen erfolgen, wodurch zwar die Spezifität erhöht wird, gleichzeitig aber die Detektionsgeschwindigkeit sinkt.

Zur Bildung des unipolaren atrialen Impedanzsignals ist die Impedanzmesseinheit vorzugsweise ausgebildet, die atriale Impedanz mit einer Abtastrate zwischen 30 Hz und 300 Hz zu erfassen. Eine geeignete Stromstärke liegt im Bereich zwischen 100 µA und 600 µA. Der zum Bestimmen der atrialen Impedanz abgegebene konstante Strom ist vorzugsweise gepulst und besitzt Stromimpulse jeweils identischer Dauer zwischen 10 µs und 20 µs. Die Stromimpulse besitzen eine paarweise alternierende Polarität und sind paarweise zu Impulspaketen zusammengefasst. Um Messartefakte weiter ausschließen zu können ist es vorteilhaft, wenn die Polaritätenfolge aufeinander folgender Stromimpulspaare alterniert.

Um die Spezifität des AF-Detektors zu erhöhen, weist die Auswerteeinheit vorzugsweise einen Wenckebach-Diskriminator auf, der ausgebildet ist, auf ein AF-Verdachtssignal anzusprechen und AF von einem AV-Block II° Typ Wenckebach zu unterscheiden und gegebenenfalls ein zuvor gebildetes AF-Verdachtssignal wieder zu löschen. Die bevorzugte Ausführungsvariante beruht auf der Erkenntnis, dass es im Falle eines AV-Blocks II° vom Typ Wenckebach auch ohne das Vorliegen von AF zum Auslöschen der Spitzenamplituden des atrialen Impedanzsignals kommen kann, wenn Abschnitte des atrialen Impedanzsignals über mehrere ventrikuläre Zyklen miteinander gemittelt werden. Dies hängt damit zusammen, dass auch im Falle eines AV-Blocks II° vom Typ Wenckebach die Synchronizität zwischen den atrialen Kontraktionen und den ventrikulären Kontraktionen verloren gehen kann. Die Mittlung des atrialen Impedanzsignals führt dazu, dass zu Ventrikelkontraktionen asynchrone Anteile des atrialen Impedanzsignals herausgemittelt werden. Im Falle eines AV-Blocks II° Typ Wenckebach kommt es infolge zunehmender Ermüdung bis zur Erschöpfung der AV-Leitung (der natürlichen Reizüberleitung vom Atrium zum Ventrikel, auch atrio-ventrikuläre Überleitung genannt) dazu, dass die Dauer eines PQ-Intervalls - also die Dauer zwischen einer atrialen Depolarisation (und Kontraktion) bis zu Beginn der ventrikulären Depolarisation und Kontraktion - periodenweise so lange zunimmt, bis eine atrio-ventrikuläre-Überleitung ausfällt. Je nachdem, ob die Zunahme der Dauer des PQ-Intervalls von Herzschlag zu Herzschlag konstant bleibt oder anwächst, werden auch die RR-Intervalle (ein RR-Intervall gibt die Dauer eines ventrikulären Zyklusses wieder) über mehrere ventrikuläre Zyklen konstant bleiben oder etwas anwachsen bis eine Überleitung ausfällt. Dadurch ergeben sich periodisch einige ventrikuläre Zyklen mit annähernd konstanten RR-Intervallen und daran anschließend ein relativ lang andauernder ventrikulärer Zyklus mit einem relativ langen RR-Intervall oder, falls ein Herzschrittmacher im Zweikammer-Modus einspringt, einem ventrikulären Zyklus mit relativ kürzerer Zyklusdauer, also kurzem RR-Intervall.

Zur Unterscheidung eines AV-Blocks II° Typ Wenckebach von AF ist daher vorzugsweise ein Wenckebach-Diskriminator vorgesehen, der ausgebildet ist, diese Unterscheidung anhand eines Stabilitäts-Kriteriums vorzunehmen. In einer bevorzugten Ausführungsvariante ermittelt der Wenckebach-Diskriminator über eine Anzahl von N aktuellen ventrikulären Zyklen jeweils kontinuierlich die mittlere Zyklendauer (das mittlere RR-Intervall) RRm oder besser noch den Meridian-Wert der RR-Intervalle. Liegt immer nur ein ventrikulärer Zyklus hinsichtlich der Dauer seines RR-Intervalls außerhalb eines vorgegebenen Stabilitäts-Bereiches, so ist dies ein Indikator, dass kein AF, sondern ein AV-Block 11° Typ Wenckebach vorliegt. Der vorgegebenen Stabilitätsbereich ist vorzugsweise durch ein auf den Mittelwert der Zyklendauer bezogenes Differenzmaß derart definiert, dass der Mittelwert der Zyklendauer RRm mit 1 plus/minus dem Differenzmaß multipliziert wird. Der Stabilitätsbereich ergibt sich also durch RRm * (1 +/- d), wobei d das Differenzmaß ist.

Liegen ab und zu auch hintereinander mindestens zwei RR-Intervalle außerhalb des so definierten Stabilitätsbereiches, schließt der Wenckebach-Diskriminator das Vorliegen eines AV-Block 11° Typ Wenckebach aus und bestätigt das AF-Verdachtssignal, das heißt der AF-Detektor detektiert dann endgültig AF.

In einer bevorzugten Ausführungsvariante ist der Wenckebach-Diskriminator ausgebildet, in der folgenden Weise zu arbeiten:

Für den Fall, dass die Spitzenamplitude des gemittelten atrialen Impedanzsignals unter den vorgegebenen Vergleichswert abgesunken ist, das heißt für den Fall, dass die Auswerteeinheit ein AF-Verdachtssignal erzeugt hat, wird der Wenckebach-Diskriminator angewandt, der auf nachfolgende

Weise einen Zählerwert bildet. Wahlweise kann dafür der Wenkebach-Dikriminator ständig im Hintergrund aktiv sein, oder nur bei Vorliegen eines AF-Verdachtssignals einen gespeicherten RR-Intervalltrend auswerten. Zunächst bildet der Wenckebach-Diskriminator für jeden ventrikulären Zyklus aktuell den Stabilitätsbereich als RR-Stabilitätsintervall durch Multiplizieren der über die aktuellen N ventrikulären Zyklen gemittelten Dauer der RR-Intervalle RRm (mittlere Zyklendauer der ventrikulären Zyklen) mit zwei Differenzfaktoren und bestimmt so die obere und untere Grenze des jeweils aktuellen RR-Stabilitätsintervalls (des aktuellen Stabilitätsbereiches). Der Differenzfaktor ergibt sich aus der Summe von 1 plus einem vorgegebenen Differenzmaß d, welches zum Beispiel 0,25 beträgt, beziehungsweise 1 minus d. Die über N ventrikuläre Zyklen gemittelte ventrikuläre Zyklendauer kann der Mittelwert der aktuellen N RR-Intervalle sein oder vorzugsweise der Median einer Anzahl N aktueller RR-Intervalle. Alternativ kann die mittlere ventrikuläre Zyklendauer auch durch einen rekursiven Filter mit N als eine Art "Zeitkonstante" gebildet werden. Ein geeigneter Wert für die Anzahl N liegt zwischen 5 und 8.

Der Wenckebach-Diskriminator prüft für jeden ventrikulären Zyklus, ob das entsprechende RR-Intervall innerhalb des auf vorstehende Weise gebildeten RR-Stabilitätsintervalls RRm * (1 +/- d) liegt. Liegt ein jeweils aktuelles RR-Intervall außerhalb dieses aktuell gültigen RR-Stabilitätsintervalls und liegt das darauffolgende RR-Intervall wieder innerhalb des RR-Stabilitätsintervalls, so wird der Zählerstand des Zählers des Wenckebach-Diskriminators um 1 hochgezählt. Der Zählerstand des Zählers des Wenckebach-Diskriminators kann dabei höchstens einen vorgegebenen Maximalwert Nmax von beispielsweise 20 erreichen, das heißt, sobald der Zählerstand 20 erreicht ist, kann dieser Zählerstand nicht weiter hochgezählt werden. Sobald zwei aufeinanderfolgende ventrikuläre Zyklen RR-Intervalle aufweisen, die außerhalb des RR-Stabilitätsintervalls liegen oder eine Mehrzahl von beispielsweise 20 aufeinanderfolgenden RR-Intervallen innerhalb des RR-Stabilitätsintervalls liegen, wird der Zähler des Wenckebach-Diskriminators wieder um 1 verkleinert. Erreicht der Zählerstand des Zählers des Wenckebach-Diskriminators eine vorgegebene Zählerstandsschwelle Nth von beispielsweise 10, detektiert der Wenckebach-Diskriminiator das Vorliegen eines AV-Block II° Typ Wenckebach und löscht das AF-Verdachtsignal. Auf diese Weise wird vermieden, dass beispielsweise eine atriale Defibrillation ausgelöst wird, obwohl keine atriale Fibrillation, sondern ein AV-Block II° Typ Wenckebach vorliegt.

Anstelle die Stabilität der RR-Intervalle auf die vorbeschriebene Art und Weise auszuwerten, kann der Wenckebach-Diskriminator auch ausgebildet sein, die Stabilität der Rate der ventrikulären Kontraktionen auszuwerten.

Die Erfindung soll nun anhand eines Ausführungsbeispiels mit bezug auf die beigefügten Figuren näher erläutert werden. Von den Figuren zeigen:
Figur 1: einen Zweikammer-Herzschrittmacher mit daran angeschlossener und im Herzen platzierter ventrikulärer und atrialer Elektrodenleitung;
Figur 2: ein schematisches Blockschaltbild des Herzschrittmachers aus Figur 1 mit einem erfindungsgemäßen AF-Detektor;
Figur 3: eine Darstellung des zum Ermitteln der unipolaren, intra-atrialen Impedanz eingesetzten, bevorzugten Messstroms; und
Figur 4: Beispiele für den Verlauf der Spitzenamplitude von gemittelten atrialen Impedanzsignalen für verschiedene Herzzustände.

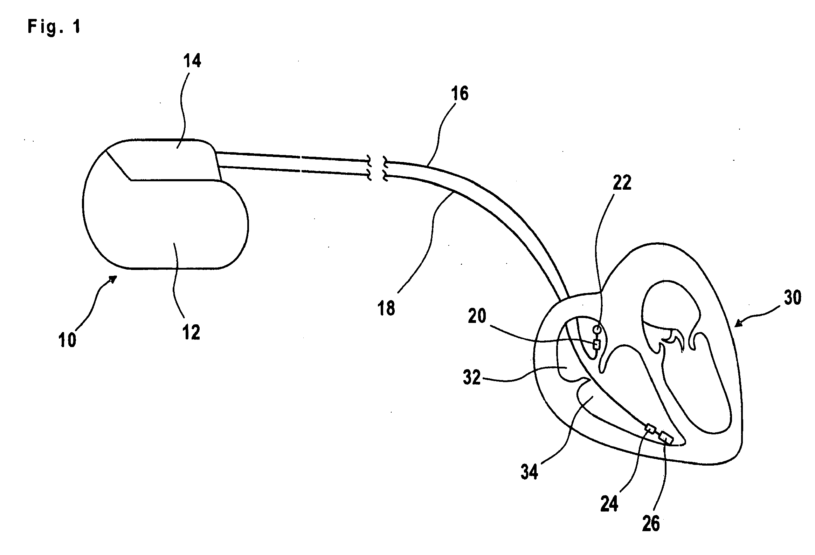

In Figur 1 ist ein implantierbarer Herzschrittmacher 10 abgebildet, der ein Hohlgehäuse 12 aus Metall besitzt sowie einen Header 14 aus transparentem, isolierendem Kunststoff, in dem Anschlüsse für Elektrodenleitungen angeordnet sind. An den Herzschrittmacher 10 sind eine atriale Elektrodenleitung 16 und eine ventrikuläre Elektrodenleitung 18 angeschlossen.

Die atriale Elektrodenleitung 16 trägt im Bereich ihres distalen Endes eine atriale Ringelektrode 20 und eine atriale Tipelektrode 22.

Die ventrikuläre Elektrodenleitung 18 trägt an ihrem distalen Ende eine ventrikuläre Ringelektrode 24 und eine ventrikuläre Tipelektrode 26.

Wie Figur 1 zu entnehmen ist, sind die distalen Enden der atrialen Elektrodenleitung 16 und der ventrikulären Elektrodenleitung 18 im Betrieb des Herzschrittmachers 10 in einem Herzen 30 platziert, und zwar derart, dass sich die atriale Ringelektrode 20 und die atriale Tipelektrode 22 im rechten Atrium 32 des Herzens 30 befinden, während die ventrikuläre Ringelektrode 24 und die ventrikuläre Tipelektrode 26 im Apex eines rechten Ventrikels 34 des Herzens 30 angeordnet sind. Für die Erfindung ist es von Vorteil, wenn wenigstens die atriale Tipelektrode 22 wandständig ist, das heißt direkt am Herzmuskelgewebe (Myokard) im Atrium 32 anliegt.

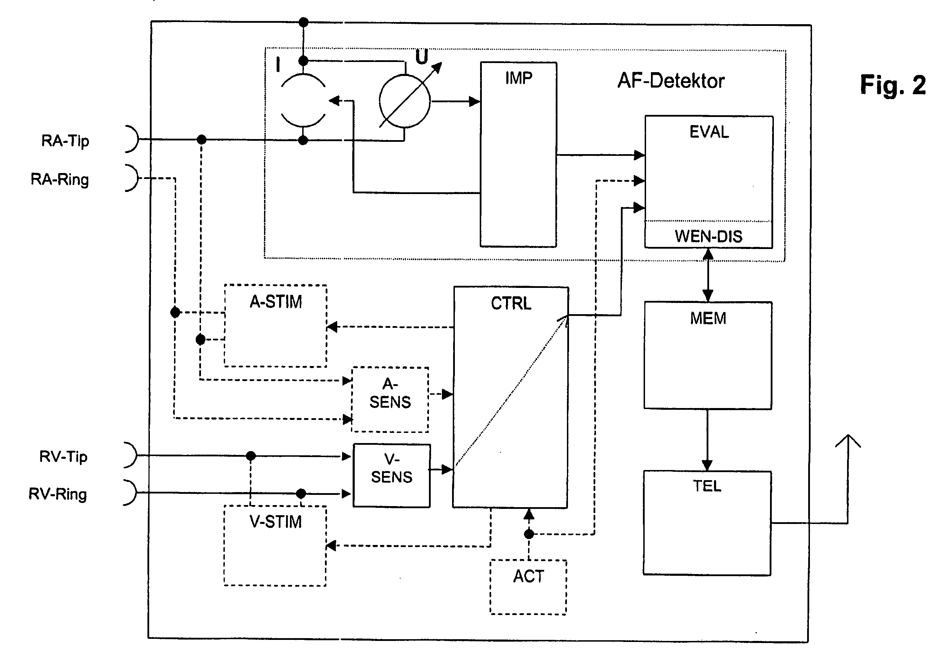

Im Inneren des Hohlgehäuses 12 des Herzschrittmachers 10 sind die wesentlichen elektrischen Komponenten des Herzschrittmachers angeordnet und über den Header 14 mit Anschlüssen für die Elektroden der atrialen Elektrodenleitung 16 und der ventrikulären Elektrodenleitung 18 elektrisch verbunden. In Figur 2 dargestellt sind ein elektrischer Anschluss RA-Ring für die rechtsatriale Ringelektrode 20, ein weiterer elektrischer Anschluss RA-Tip für die rechtsatroiale Ringelektrode 22, ein elektrischer Anschluss RV-Ring für die rechtsventrikuläre Ringelektrode 24 und ein elektrischer Anschluss RV-Tip für die rechtsventrikuläre Tipelektrode 26.

In einer für einen Zweikammer-Herzschrittmacher üblichen Manier sind die Anschlüsse RA-Ring und RA-Tip mit einer atrialen Stimulationseinheit A-STIM und einer atrialen Sensing-Einheit A-SENS und die Anschlüsse RV-Ring und RV-Tip mit einer ventrikulären Stimulationseinheit V-STIM und einer ventrikulären Sensing-Einheit V-SENS verbunden. Die Stimulations- und Sensing-Einheiten sind jeweils mit einer zentralen Steuereinheit CTRL verbunden und erlauben mit dieser zusammen die bedarfsabhängige Stimulation in einem Demand-Modus wie beispielsweise DDD.

Für die vorliegende Erfindung von vorrangiger Bedeutung ist ein AF-Detektor für die Detektion von atrialer Fibrillation oder Flatterns (AF). Der AF-Detektor umfasst mehrere Bestandteile, die in Figur 1 durch eine punktierte Linie eingerahmt sind. Zu diesen Bestandteilen zählt eine Impedanzbestimmungseinheit IMP, die mit einer Konstantstromquelle I und einer Spannungsmesseinheit U verbunden ist. Die Impedanzbestimmungseinheit IMP ergibt zusammen mit der Konstantstromquelle I und der Spannungsmesseinheit U eine Impedanzmesseinheit.

Weiterer Bestandteil des AF-Detektors ist eine Auswerteeinheit EVAL die ihrerseits als Bestandteil einen Wenckebach-Diskriminator WEN-DIS aufweist. Nicht näher dargestellt ist ein Zähler als Bestandteil des Wenckebach-Diskriminators WEN-DIS.

Die Konstantstromquelle I der Impedanzmesseinheit ist einerseits mit dem Anschluss RA-Tip für die rechtsatriale Tipelektrode 22 und andererseits mit dem metallischen Hohlgehäuse 12 des Herzschrittmachers 10 als Neutralelektrode verbunden. Gleiches gilt für die Spannungsmesseinheit U, die ausgebildet ist, den Spannungsabfall zu messen, der über diese beiden Elektroden anliegt, wenn die Konstantstromquelle I einen Messstrom zum Messen der intraatrialen Impedanz abgibt.

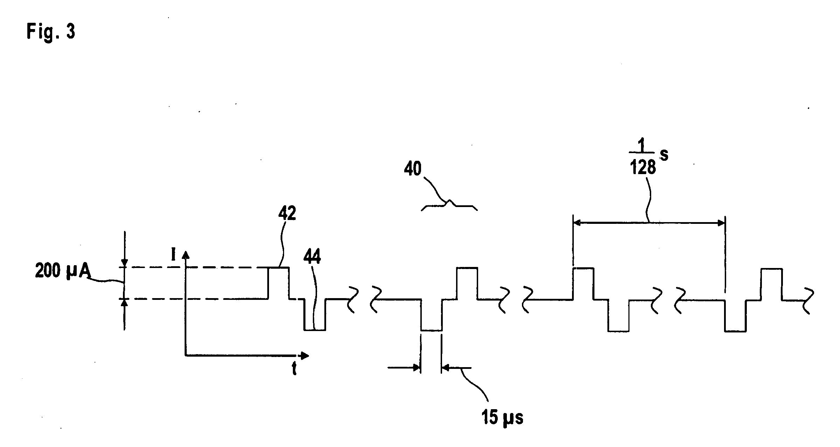

Die Konstantstromquelle I ist ausgebildet, einen Messstrom der in Figur 3 skizzenhaft dargestellten Art zu generieren und abzugeben. 128 Mal pro Sekunde erzeugt die Konstantstromquelle I ein Stromimpulspaar 40 von insgesamt 45 µs Dauer, welches jeweils von zwei, jeweils 15µs dauernden Stromimpulsen 42 und 44 gleicher absoluter Stromstärke aber gegensätzlicher Polarität gebildet wird. Zwischen den beiden Stromimpulsen 42 und 44 eines Stromimpulspaares 40 ist jeweils eine Pause von ebenfalls 15 µs Dauer vorgesehen. Die Stromimpulspaare 40 wiederholen sich alle 128tel Sekunde. Dabei wechselt, wie in Figur 3 dargestellt, die Polaritätenfolge von Stromimpulspaar zu Stromimpulspaar, das heißt ein erstes Stromimpulspaar beginnt mit einem positiven Stromimpuls 42 und endet mit einem negativen Stromimpuls 44, während das darauffolgende Stromimpulspaar 40 mit einem negativen Stromimpuls beginnt und mit einem positiven Stromimpuls endet. Auf diese Weise wird die Bildung von Artefakten vermieden.

Wie sich aus dem vorstehenden ergibt, wird die atriale Impedanz mit einer Abtastrate von 128 Hz abgetastet. Die Impedanzbestimmungseinheit IMP bildet aus den Werten für die Stromstärke und die zugehörige Messspannung durch Quotientenbildung jeweils einen Impedanzwert und erzeugt auf diese Weise ein unipolares atriales Impedanzsignal, welches von der Impedanzbestimmungseinheit IMP der Auswerteeinheit EVAL zugeführt wird. In der Auswerteeinheit EVAL wird das atriale Impedanzsignal in der zuvor beschriebenen Weise ausgewertet. Um diese Auswertung vornehmen zu können, wird der Auswerteeinheit EVAL außerdem ein Ventrikelsignal zugeführt. Dieses Ventrikelsignal repräsentiert die Zeitpunkte ventrikulärer Kontraktionen in zeitlicher Zuordnung zu dem atrialen Impedanzsignal.

Das Ventrikelsignal wird in an sich bekannter Weise mit Hilfe der Steuereinheit CTRL des Herzschrittmachers gebildet, die mit der ventrikulären Sensingstufe V-SENS verbunden ist. Die ventrikuläre Sensingstufe ist ihrerseits im Betrieb mit der ventrikulären Ringelektrode 24 und der ventrikulären Tipelektrode 26 über entsprechende Anschlüsse RV-Ring und RV-Tip des Herzschrittmachers 10 verbunden.

Wie eingangs bereits beschrieben, bildet die Auswerteeinheit EVAL zwischen jeweils zwei aufeinanderfolgenden ventrikulären Kontraktionen liegende Signalabschnitte des atrialen Impedanzsignals und mittelt diese Signalabschnitte über jeweils acht aktuelle ventrikuläre Zyklen.

Bei gesundem Herzen erfolgt eine jeweilige ventrikuläre Kontraktion nach einer relativ konstanten atrio-ventrikulären Überleitungszeit auf eine atriale Kontraktion, so dass eine Synchronizität zwischen atrialen und ventrikulären Kontraktionen besteht. Das auf die vorbeschriebene Art und Weise gemittelte atriale Impedanzsignal gibt in diesem gesunden Fall den typischen Verlauf der atrialen Impedanz zwischen zwei aufeinanderfolgenden ventrikulären Kontraktionen wieder und besitzt ein Amplitudenmaximum zu einem Zeitpunkt, an dem die atriale Kontraktion stattfindet. Dieser Zeitpunkt liegt vor einer jeweiligen, darauffolgenden ventrikulären Kontraktion und hat zu dieser einen zeitlichen Abstand, der in etwa der atrio-ventrikulären Überleitungszeit entspricht.

Im Falle von AF, aber auch im Falle eines AV-Blocks II° vom Typ Wenckebach besteht die Synchronizität zwischen atrialen Kontraktionen und ventrikulären Kontraktionen nicht. Dies führt dazu, dass sich die typischen Maximalamplituden des Verlaufs der atrialen Impedanz zwischen zwei ventrikulären Kontraktionen bei Mittlung über mehrere ventrikuläre Zyklen ausmitteln, da sie in bezug auf einen jeweiligen ventrikulären Zyklus zu unterschiedlichen Zeitpunkten auftreten.

Die Erfindung macht sich diesen Sachverhalt zunutze, indem die Auswerteeinheit EVAL eine nicht vorhandene, atrio-ventrikuläre Synchronizität durch Auswerten des gemittelten atrialen Impedanzsignals detektiert. Hierzu wird die Spitzenamplitude des gemittelten atrialen Impedanzsignals mit einem Vergleichswert verglichen und eine nicht vorhandene atrio-ventrikuläre Synchronizität dann detektiert, wenn die Spitzenamplitude des atrialen Impedanzsignals geringer ist als der Vergleichswert. Da eine nicht vorhandene atrio-ventrikuläre Synchronizität ihre Ursache auch in einem AV-Block II° Typ Wenckebach haben kann, besitzt die Auswerteeinheit EVAL in ihrer hier dargestellten, bevorzugten Ausführungsvariante außerdem noch einen Wenckebach-Diskriminator WEN-DIS, der ausgebildet ist, auf die eingangs beschriebene Art und Weise das Vorhandensein eines AV-Blocks II° vom Typ Wenckebach zu erfassen, falls die Auswerteeinheit EVAL zunächst eine mangelnde atrio-ventrikuläre Synchronizität festgestellt und daraufhin ein AV-Verdachtssignal erzeugt hat. Der Wenckebach-Diskriminator WEN-DIS wirkt als eine Art Filter im Ausgang der Auswerteeinheit EVAL und sorgt dafür, dass die Auswerteeinheit EVAL nur dann ein AF-Verdachtssignal ausgibt, wenn die Auswerteeinheit zum einen die mangelnde atrio-ventrikuläre Synchronizität festgestellt und der Wenckebach-Diskriminator zum anderen das Nichtvorhandensein eines AV-Blocks II° vom Typ Wenckebach festgestellt hat.

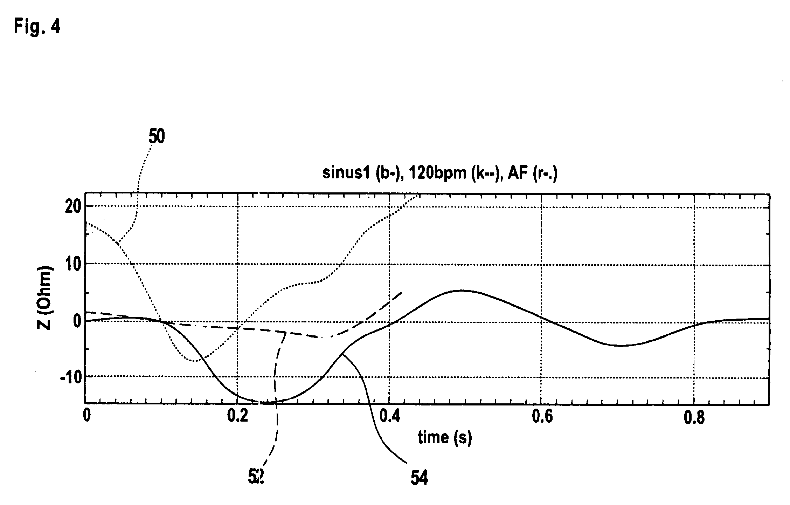

Zur Erläuterung der Arbeitsweise der Auswerteeinheit EVAL sind in Figur 4 drei verschiedene intraatriale Impedanzverläufe dargestellt, nämlich für den Fall des gesunden Herzens (Kurve 50), für den Fall des stimulierten Herzens (Kurve 52) und für den Fall des Vorliegens von AF (Kurve 54). Deutlich ist zu erkennen, dass die Maximalamplitude des gemittelten intraatrialen Impedanzsignals im Falle einer AF vergleichsweise sehr klein ist.

Für langzeitdiagnostische Zwecke werden die Phasen des Vorliegens eines AF-Verdachtssignals in einem Speicher MEM gespeichert und können mittels einer Telemetrieeinheit TEL drahtlos beispielsweise an ein Service Center übermittelt werden.

Darüber hinaus besitzt der Herzschrittmacher die üblichen Bestandteile zur ratenadaptiven Stimulation des Ventrikels und des Atriums wie beispielsweise die Stimulationseinheiten V-STIM und A-STIM, die Sensing-Einheiten V-SENS und A-SENS, die Steuereinheit CTRL und einen Aktivitätssensor ACT, der es erlaubt, die jeweilige Stimulationsrate an den physiologischen Bedarf eines Patienten anzupassen.

Außerdem kann der Herzschrittmacher auch als Kardioverter/Defibrillator insbesondere als atrialer Defibrillator ausgebildet sein und solche für diesen Zweck angepasste atriale Stimulationseinheiten aufweisen, wie sie aus dem Stand der Technik grundsätzlich bekannt sind.

## Patentansprüche

1. Detektor für atriales Flimmern oder Flattern (AF)
mit einer Impedanzmesseinheit, die einen Messeingang aufweist, an den eine atriale Elektrodenleitung mit einer Elektrode für eine unipolare Messung einer Impedanz im Atrium aufweist und ausgebildet ist, ein unipolar gewonnenes atriales Impedanzsignal derart zu generieren, dass das Impedanzsignal für jeden, eine atriale Kontraktion und die darauf folgende Entspannung des Atriums umfassenden atrialen Zyklus mehrere, zu verschiedenen Zeitpunkten innerhalb eines jeweiligen atrialen Zyklusses erfasste Impedanzwerte umfasst, und
mit einem Signaleingang, über den dem Detektor ein Ventrikelsignal zuzuführen ist, welches die Zeitpunkte ventrikulärer Kontraktionen in zeitlicher Zuordnung zu dem Impedanzsignal widerspiegelt,
wobei der Detektor eine Auswerteeinheit aufweist, die ausgebildet ist,
mehrere aufeinanderfolgende, von jeweils zwei aufeinanderfolgenden ventrikulären Kontraktionen begrenzte Impedanzsignalabschnitte eines unipolaren atrialen Impedanzsignals miteinander zu mitteln und
die maximale Amplitude des gemittelten unipolaren atrialen Impedanzsignalabschnitts zu bestimmen,
mit einem Vergleichswert zu vergleichen und
im Falle, dass die maximale Amplitude des gemittelten unipolaren atrialen Impedanzsignals geringer ist, als der Vergleichswert, ein AF-Verdachtssignal zu erzeugen.

2. Detektor nach Anspruch 1, **dadurch gekennzeichnet, dass** die Auswerteeinheit ausgebildet ist, immer die jeweils acht aktuellsten Impedanzsignalabschnitte miteinander zu mitteln.

3. Detektor nach Anspruch 1 oder 2, **dadurch gekennzeichnet, dass** die Impedanzmesseinheit ausgebildet ist, die unipolare, intrakardiale Impedanz mit einer Abtastrate zwischen 30Hz und 300Hz zu erfassen.

4. Detektor nach einem der Ansprüche 1 bis 3, **dadurch gekennzeichnet, dass** die Impedanzmesseinheit ausgebildet ist, zur Messung der unipolaren, intrakardialen Impedanz Stromimpulse mit einer Stromstärke zwischen 100µA und 600µA zu erzeugen und über eine Neutralelektrode eine atriale Elektrode abzugeben und den während der Abgabe der Stromimpulse über diese beiden Elektroden auftretenden Spannungsabfall zu messen.

5. Detektor nach Anspruch 4, **dadurch gekennzeichnet, dass** die Impedanzmesseinheit ausgebildet ist, Stromimpulse einer jeweils identischen Dauer zwischen 10µs und 20µs abzugeben.

6. Detektor nach Anspruch 5, **dadurch gekennzeichnet, dass** die Impedanzmesseinheit ausgebildet ist, paarweise jeweils zwei Stromimpulse gleicher Dauer und gleicher absoluter Stromstärke aber unterschiedlicher Polarität abzugeben.

7. Detektor nach Anspruch 6, **dadurch gekennzeichnet, dass** die Impedanzmesseinheit derart ausgebildet ist, dass die Polaritätenfolge aufeinanderfolgender Stromimpulspaare alterniert.

8. Detektor nach einem der Ansprüche 1 bis 7, **dadurch gekennzeichnet, dass** die Auswerteeinheit einen Wenckebach-Diskrimator aufweist, der ausgebindet ist, auf ein AF-Verdachtssignal anzusprechen und AF von einem AV-Block II Typ Wenckebach zu unterschieden und gegebenenfalls das AF-Verdachtssignal zu löschen.

9. Detektor nach Anspruch 8, **dadurch gekennzeichnet, dass** der Wenckebach-Diskriminator ausgebildet ist anhand des Ventrikelsignals einen Mittelwert der Zyklendauer eines ventrikulären Zyklusses (RR-Intervall) über eine vorgegebene Anzahl ventrikulärer Zyklen zu bilden, und im Falle des Vorliegens des AF-Verdachtssignals die Dauer eines aktuellen ventrikulären Zyklusses mit dem Mittelwert der Zyklendauer zu vergleichen und zu prüfen, ob jeweils nur ein vereinzelter ventrikulärer Zyklus oder mehrere aufeinanderfolgende ventrikuläre Zyklen hinsichtlich ihrer Dauer um mehr als ein vorgegebenes Differenzmaß von dem Mittelwert der Zyklendauer abweichen und im ersten Fall das AF-Verdachtssignal wieder zu löschen.

10. Detektor nach Anspruch 9, **dadurch gekennzeichnet, dass** der Wenckebach-Diskriminator ausgebildet ist, das Differenzmaß aus einem über eine vorgegebene Anzahl aktueller ventrikulärer Zyklen gebildeten Mittelwert der Zyklendauer ventrikulärer Zyklen durch Multiplizieren dieses Mittelwertes mit einem vorgegebenen konstanten Faktor zu bilden.

11. Detektor nach Anspruch 9 oder 10, **dadurch gekennzeichnet, dass** der Wenckebach-Diskriminator ausgebildet ist, für jeden ventrikulären Zyklus, in dem ein einzelner ventrikulärer Zyklus hinsichtlich seiner Dauer um mehr als ein vorgegebenes Differenzmaß von dem Mittelwert der Zyklendauer abweicht, ein Wenckebach-Signal zu erzeugen.

12. Detektor nach Anspruch 11, **dadurch gekennzeichnet, dass** der Wenckebach-Diskriminator einen Zähler aufweist, der ausgebildet ist, seinen Zählerstand um 1 zu erhöhen, falls der Wenckebach-Diskriminator für einen vereinzelten aktuellen ventrikulären Zyklus ein Wenckebach-Signal erzeugt hat.

13. Detektor nach Anspruch 12, **dadurch gekennzeichnet, dass** der Zähler ausgebildet ist, seinen Zählerstand um 1 zu verringern, falls der Wenckebach-Diskriminator detektiert hat, dass zwei aktuelle, aufeinanderfolgende ventrikuläre Zyklen um mehr als ein vorgegebenes Differenzmaß von dem Mittelwert der Zyklendauer abweichen oder für eine Mehrzahl von wenigstens fünf aufeinanderfolgenden, aktuellen ventrikulären Zyklen kein Wenckebach-Signal erzeugt hat.

14. Detektor nach Anspruch 13, **dadurch gekennzeichnet, dass** die Auswerteeinheit ausgebildet ist, das AF-Verdachtssignal zu löschen und den Zähler zurückzusetzen, wenn der Zählerstand eine vorgegebene Zählerstandschwelle überschreitet.

## Claims

1. A detector for atrial fibrillation or flutter (AF),
comprising an impedance measuring unit having a measurement input on which an atrial electrode line with an electrode for a unipolar measurement of an impedance in the atrium is formed, to generate a unipolar atrial impedance signal, such that the impedance signal has several impedance values detected at different points in time within a respective atrial cycle for each atrial cycle comprising an atrial contraction and the successive relaxation of the atrium, and
comprising a signal input by which a ventricular signal is to be supplied to the detector, said signal reflecting the points in time of ventricular contractions in chronological correlation with the impedance signal,
such that the detector has an evaluation unit designed
to average several successive impedance signal sections of a unipolar atrial impedance signal that are bordered by two successive ventricular contractions, and
to determine the maximum amplitude of the average unipolar atrial impedance signal section,
to compare it with a reference value, and
in the event the maximum amplitude of the average unipolar atrial impedance signal is lower than the reference value, to generate a suspected AF signal.

2. The detector according to Claim 1,
**characterized in that**
the evaluation unit is designed to always average the eight most recent impedance signal sections.

3. The detector according to Claim 1 or 2,
**characterized in that**
the impedance measuring unit is designed to detect the unipolar intracardiac impedance with a scanning rate between 30 Hz and 300 Hz.

4. The detector according to any one of Claims 1 to 3,
**characterized in that**
the impedance measuring unit is designed to generate current pulses with amperage between 100 µA and 600 pA for measurement of the unipolar intracardiac impedance and to deliver them to an atrial electrode via a neutral electrode and to measure the voltage drop occurring across these two electrodes during the delivery of the current pulses.

5. The detector according to Claim 4,
**characterized in that**
the impedance measuring unit is designed to deliver current pulses of an identical duration between 10 µs and 20 µs.

6. The detector according to Claim 5,
**characterized in that**
the impedance measuring unit is designed to deliver two current pulses of the same duration and the same absolute amperage but different polarities in pairs.

7. The detector according to Claim 6,
**characterized in that**
the impedance measuring unit is designed so that the polarity sequence of successive current pulse pairs alternates.

8. The detector according to any one of Claims 1 to 7,
**characterized in that**
the evaluation unit has a Wenckebach discriminator designed to respond to a suspected AF signal and to differentiate AF from a second-degree AV block of the Wenckebach type and optionally to delete the suspected AF signal if necessary.

9. The detector according to Claim 8,
**characterized in that**
the Wenckebach discriminator is designed to form an average of the cycle duration of a ventricular cycle (RR interval) over a predefined number of ventricular cycles on the basis of the ventricular signal and, in the event of a suspected AF signal, to compare the duration of a current ventricular cycle with the average of the cycle duration and to check on whether only one isolated ventricular cycle or several successive ventricular cycles deviate from the average of the cycle duration by more than a predefined difference measure and to delete the suspected AF signal in the former case.

10. The detector according to Claim 9,
**characterized in that**
the Wenckebach discriminator is designed to form the difference measure from an average of the cycle duration of ventricular cycles formed over a predefined number of current ventricular cycles by multiplying this average times a predefined constant factor.

11. The detector according to Claim 9 or 10,
**characterized in that**
the Wenckebach discriminator is designed to generate a Wenckebach signal for each ventricular cycle in which a single ventricular cycle differs with regard to its duration from the average of the cycle duration by more than a predefined difference measure.

12. The detector according to Claim 11,
**characterized in that**
the Wenckebach discriminator has a counter designed to increment its counter reading by 1 if the Wenckebach discriminator has generated a Wenckebach signal for an isolated current ventricular cycle.

13. The detector according to Claim 12,
**characterized in that**
the counter is designed to decrement its counter reading by 1 if the Wenckebach discriminator has detected that two current successive ventricular cycles deviate from the average of the cycled duration by more than a predefined difference measure or no Wenckebach signal has been generated for a plurality of at least five successive current ventricular cycles.

14. The detector according to Claim 13,
**characterized in that**
the evaluation unit is designed to delete the suspected AF signal and reset the counter when the counter reading exceeds a predefined counter reading threshold.

## Revendications

1. Détecteur pour un scintillement ou un papillotement (AF) atrial, comprenant une unité de mesure d'impédance comportant une entrée de mesure qui présente une ligne d'électrodes atriale avec une électrode pour une mesure unipolaire d'une impédance dans l'atrium et est conçue pour générer un signal d'impédance atrial obtenu de façon unipolaire de telle sorte que le signal d'impédance comporte pour chaque cycle atrial comportant une contraction atriale et la détente consécutive de l'atrium plusieurs valeurs d'impédance enregistrées à différents moments à l'intérieur d'un cycle atrial respectif, et
une entrée de signal par laquelle un signal de ventricule doit être amené au détecteur, lequel signal reflète les moments de contractions ventriculaires dans une attribution temporelle au signal d'impédance,
le détecteur présentant une unité d'évaluation qui est conçue pour
faire la moyenne de plusieurs parties de signal d'impédance, successives, limitées par respectivement deux contractions ventriculaires consécutives, d'un signal d'impédance atrial unipolaire et
déterminer l'amplitude maximale de la partie de signal d'impédance atriale unipolaire moyenne,
la comparer avec une valeur de comparaison et,
dans le cas où l'amplitude maximale du signal d'impédance atrial unipolaire moyen est plus faible que la valeur de comparaison, générer un signal de soupçon de AF.

2. Détecteur selon la revendication 1, **caractérisé en ce que** l'unité d'évaluation est conçue pour déterminer la moyenne toujours pour les respectivement huit parties de signal d'impédance les plus actuelles.

3. Détecteur selon la revendication 1 ou 2, **caractérisé en ce que** l'unité de mesure d'impédance est conçue pour enregistrer l'impédance unipolaire et intracardiaque avec un taux de balayage compris entre 30Hz et 300Hz.

4. Détecteur selon l'une quelconque des revendications 1 à 3, **caractérisé en ce que** l'unité de mesure d'impédance est conçue pour générer des impulsions de courant avec une intensité de courant comprise entre 100µA et 600µA pour la mesure de l'impédance unipolaire et intracardiaque et mesurer la chute de tension qui apparaît sur ces deux électrodes pendant la délivrance des impulsions de courant.

5. Détecteur selon la revendication 4, **caractérisé en ce que** l'unité de mesure d'impédance est conçue pour délivrer des impulsions de courant d'une durée respectivement identique comprise entre 10µ et 20µs.

6. Détecteur selon la revendication 5, **caractérisé en ce que** l'unité de mesure d'impédance est conçue pour délivrer par paires à chaque fois deux impulsions de courant de même durée et de même intensité de courant absolue, mais de polarité différente.

7. Détecteur selon la revendication 6, **caractérisé en ce que** l'unité de mesure de mesure d'impédance est conçue de telle sorte que la succession de polarités alterne des paires d'impulsions de courant successives.

8. Détecteur selon l'une quelconque des revendications 1 à 7, **caractérisé en ce que** l'unité d'évaluation présente un discriminateur de Wenckebach qui est conçu pour réagir à un signal de présomption de AF et différencier des AF d'un bloc AV Il type Wenckebach et éventuellement effacer le signal de présomption de AF.

9. Détecteur selon la revendication 8, **caractérisé en ce que** le discriminateur de Wenckebach est conçu pour former à l'aide du signal de ventricule une valeur moyenne de la durée de cycle d'un cycle ventriculaire (intervalle RR) pour un nombre prédéfini de cycles ventriculaires, et, dans le cas de la présence du signal de présomption de AF, de comparer la durée d'un cycle ventriculaire actuelle avec la valeur moyenne de la durée de cycle et de vérifier si à chaque fois un cycle ventriculaire individuel ou plusieurs cycles ventriculaires successifs diffèrent en ce qui concerne leur durée de plus d'une cote de différence prédéfinie par rapport à la valeur moyenne de la durée de cycle et effacer à nouveau le signal de présomption de AF dans le premier cas.

10. Détecteur selon la revendication 9, **caractérisé en ce que** le discriminateur de Wenckebach est conçu pour former la cote de différence à partir d'une valeur moyenne, formée pour un nombre prédéfini de cycles ventriculaires actuels, de la durée de cycle de cycles ventriculaires par la multiplication de cette valeur moyenne par un facteur constant prédéfini.

11. Détecteur selon la revendication 9 ou 10, **caractérisé en ce que** le discriminateur de Wenckebach est conçu pour générer un signal de Wenckebach pour chaque cycle ventriculaire dans lequel un cycle ventriculaire individuel diffère en ce qui concerne sa durée de plus d'une cote de différence prédéfinie par rapport à la valeur moyenne de la durée de cycle.

12. Détecteur selon la revendication 11, **caractérisé en ce que** le détecteur de Wenckebach présente un compteur qui est conçu pour augmenter son indication de compteur de 1 dans le cas où le discriminateur de Wenckebach a généré un signal de Wenckebach pour un cycle ventriculaire actuel individuel.

13. Détecteur selon la revendication 12, **caractérisé en ce que** le compteur est conçu pour réduire son indication de compteur de 1 dans le cas où le discriminateur de Wenckebach a détecté que deux cycles ventriculaires actuels et consécutifs diffèrent de plus d'une cote de différence prédéfinie par rapport à la valeur moyenne de la durée de cycle ou n'a pas généré de signal de Wenckebach pour une pluralité d'au moins cinq cycles ventriculaires actuels et consécutifs.

14. Détecteur selon la revendication 13, **caractérisé en ce que** l'unité d'évaluation est conçue pour effacer le signal de présomption de AF et remettre le compteur à zéro lorsque l'indication du compteur dépasse un seuil d'indication de compteur prédéfini.
